Europäisches Patentamt

European Patent Office

Office européen des brevets

(19)

(11) Numéro de publication : **0 470 027 A1**

# (12) DEMANDE DE BREVET EUROPEEN

(21) Numéro de dépôt : **91460039.0**

(22) Date de dépôt : **22.07.91**

(51) Int. Cl.$^5$ : **C12N 1/06, A23K 1/00, A23J 1/18**

(30) Priorité : **30.07.90 FR 9009874**

(43) Date de publication de la demande :
**05.02.92 Bulletin 92/06**

(84) Etats contractants désignés :
**AT BE CH DE DK ES GB GR IT LI LU NL SE**

(71) Demandeur : **Cohas, Pascal**
**27, Allée Gabriel Andouard**
**F-53000 Laval (FR)**
Demandeur : **Cohas, Patrice**
**Rue de la Motte**
**F-35630 Hede (FR)**

(72) Inventeur : **Cohas, Pascal**
**27, Allée Gabriel Andouard**
**F-53000 Laval (FR)**
Inventeur : **Cohas, Patrice**
**Rue de la Motte**
**F-35630 Hede (FR)**

(74) Mandataire : **Le Guen, Louis François**
**CABINET Louis LE GUEN 38, rue Levavasseur**
**B.P. 91**
**F-35802 Dinard Cédex (FR)**

(54) **Procédé de préparation de lysat de levures en suspension.**

(57) L'invention concerne un procédé de préparation de lysat de levures ou de levures lactiques ou d'un mélange de ces levures en suspension. Il consiste à brasser le lysat en préparation et à le stabiliser en ajoutant de 2,6 à 10 % d'un sel ou d'un mélange de sels phosphoriques ou phosphoreux, ou d'acide ou d'anhydride phosphorique ou phosphoreux. On ajoute, avant brassage, ou avant ou après stabilisation, 1 à 20 % de vitamine $B_T$ qui augmente l'activité des levures lors de cette préparation.

EP 0 470 027 A1

La présente invention concerne un procédé de préparation de lysats de levures ou de levures lactiques, ou d'un mélange de ces levures en suspension, lesdits lysats étant enrichis en vitamine $B_T$.

Les lysats de levures sont utilisables, en particulier, comme additifs dans l'alimentation de nombreux animaux chez qui ils sont un facteur de croissance et ont un effet régulateur sur la flore bactérienne intestinale.

Le procédé de préparation suivant l'invention a pour objet d'obtenir de tels lysats en partant aussi bien de la levure de bière que de la levure de panification ou des sous-produits de ces levures.

On connaît déjà, par exemple par la demande de brevet n° 89 04341 aux noms des présents demandeurs, un tel procédé de préparation. Il consiste à chauffer les levures à une température de 72° C pendant au moins 20 minutes en présence de chlorure de sodium puis, une fois les cellules de levure éclatées, à les additionner à un sel ou à un mélange de sels ou d'acides ou d'anhydrides.

On a cherché à augmenter l'activité des levures utilisées lors de cette préparation et on a trouvé qu'on pouvait le faire par apport de vitamine $B_T$.

Ainsi, suivant une caractéristique de l'invention, il est prévu un procédé de préparation de lysats en suspension de levures ou de levures lactiques ou un mélange de levures dans lequel on ajoute de 1 à 20% de vitamine $B_T$, puis on brasse le lysat en suspension.

Pour terminer la préparation, on ajoute sous agitation de 0,1 à 5 % de sulfate de fer et de 2,6 à 10 % d'un sel ou d'un mélange de sels phosphoriques ou phosphoreux, ou d'acide ou d'anhydride phosphorique ou phosphoreux.

On a, par cette dernière étape du procédé, fait subir aux levures un traitement de stabilisation évitant une éventuelle fermentation.

La vitamine $B_T$ peut être ajoutée, non avant l'étape de brassage mais après, et avant ou après le traitement de stabilisation.

Suivant une autre caractéristique, lesdits sels phosphoriques ou phosphoreux sont des sels non organiques.

Suivant une autre caractéristique de l'invention on ajoute de 2,6 à 10 % de chlorure de sodium.

Dans un exemple d'application du procédé suivant l'invention, on a fabriqué 2000 kgs de lysat de levures en suspension enrichi en vitamine $B_T$ en:

a) mélangeant:

825 kgs de levure de boulangerie,
1000 kgs de levure de bière,
40 kgs de vitamine $B_T$, et
55 kgs de chlorure de sodium.

b) brassant le mélange, et

c) ajoutant 75 kgs de sels phosphoriques et 5 kgs de sulfate de fer.

On obtient alors un lysat en suspension dont les caractéristiques au litre sont les suivantes:

– Taux de protéines (N x 6,25) 10 à 15 %
– Taux de matières sèches 20 à 34 %
– Poids spécifique 1,050 à 1,150
– Nombre de cellules revivifiables au gramme moins de 10 000

On peut transformer ce lysat humide en poudre par séchage en utilisant, par exemple, un séchoir à billes, à turbine ou à buse, ou tout autre mode de séchage.

Le produit en poudre obtenu a alors les caractéristiques suivantes:

– Taux de protéines (N x 6,25) 32 à 48 %
– Taux de matières sèches 88 à 98 %
– Nombre de cellules revivifiables au gramme moins de 10 000

Dans le cas où l'on transforme le lysat en suspension sous forme de poudre, il est possible de diminuer l'apport phosphorique.

Les quantités données dans l'application particulière ci-dessus le sont à titre d'exemple. Elles peuvent être différentes dès lors qu'elles entrent dans les fourchettes caractéristiques du procédé.

Les quantités de chlorure de sodium et de sulfate de fer peuvent être modifiées, voire, annulées. Dans ce dernier cas, les caractéristiques du lysat obtenu sont encore bonnes.

## Revendications

1) Procédé de préparation de lysat de levures ou de levures lactiques ou d'un mélange de ces levures en suspension, caractérisé en ce qu'on brasse le lysat en préparation, puis on stabilise le mélange en ajoutant de 2,6 à 10 % d'un sel ou d'un mélange de sels phosphoriques ou phosphoreux, ou d'acide ou d'anhydride phosphorique ou phosphoreux, 1 à 20 % de vitamine $B_T$ étant ajoutée avant brassage, avant stabilisation ou après stabilisation.

2) Procédé suivant la revendication 1, caractérisé en ce que lesdits sels phosphoriques ou phosphoreux utilisés sont des sels minéraux.

3) Procédé selon la revendication 1 ou 2, caractérisé en ce qu'en même temps qu'on ajoute au lysat la vitamine $B_T$, on ajoute de 2,6 à 10 % de chlorure de sodium.

4) Procédé selon une des revendications précédentes, caractérisé en ce qu'en même temps qu'on ajoute un sel ou une mélange de sels phosphoriques ou phosphoreux, on ajoute de 0,1 à 5 % de sulfate de fer.

5) Procédé selon la revendication 4, caractérisé en ce que, pour fabriquer 2000 kg de lysat de levures:

a) on mélange 825 kgs de levure de boulangerie, 1000 kgs de levure de bière, 40 kgs de vitamine $B_T$, et 55 kgs de chlorure de sodium.

b) on brasse le mélange obtenu, et

c) on ajoute 75 kgs de sels phosphoriques et 5 kgs de sulfate de fer.

**6)** Procédé selon la revendication 5, caractérisé en ce que, dans l'étape <u>a</u>, on omet d'ajouter le chlorure de sodium.

**7)** Procédé selon la revendication 5 ou 6, caractérisé en ce que, dans l'étape <u>c</u>, on omet d'ajouter le sulfate de fer.

**Office européen des brevets**

# RAPPORT DE RECHERCHE EUROPEENNE

Numero de la demande

EP 91 46 0039

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | GB-A- 952 713 (SCHWARZ BIO RESEARCH INC.) * Page 1, lignes 56-88; page 2, lignes 1-27; exemples 1-6 * --- | 1,5 | C 12 N 1/06 A 23 K 1/00 A 23 J 1/18 |
| A | EP-A-0 234 863 (BOVRIL LTD) * Revendications 1-4; page 4 * --- | 1 | |
| A | WPIL/DERWENT, accession nr. 89-181230 [25], Derwent Publications Ltd, Londres, GB; & JP-A-1 117 794 (AJINOMOTO K.K.) 10-05-1989 --- | | |
| A | EP-A-0 039 415 (SOCIETE DES PRODUITS NESTLE S.A.) ----- | | |

**DOMAINES TECHNIQUES RECHERCHES (Int. Cl.5)**

C 12 N

Le présent rapport a été établi pour toutes les revendications

| Lieu de la recherche | Date d'achèvement de la recherche | Examinateur |
|---|---|---|
| LA HAYE | 18-10-1991 | SANTOS Y DIAZ A.I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant